# EUROPEAN PATENT SPECIFICATION

(11) **EP 1 614 437 B1**
(45) Date of publication and mention of the grant of the patent: **31.03.2010**
(21) Application number: 04380139.8
(22) Date of filing: 08.07.2004
(51) Int. Cl.: A61M 1/34, A61M 1/16, C02F 1/44, B01D 61/10, B01D 61/04

(54) **Water treatment system for haemodialysis**
System zur Vorbehandlung von Wasser für die Hämodialyse
Systeme de pretraitement de l'eau pour l'hemodialyse

(43) Date of publication of application: 11.01.2006
(73) Proprietor: Peter Taboada, S.l., 36800 Redondela, Pontevedra (ES)
(72) Inventor: Taboada Presedo, Jesus Manuel, 36800 Redondela Pontevedra (ES)
(74) Representative: Temino Ceniceros, Ignacio

(56) References cited:
- EP-A- 0 752 391
- EP-A- 1 234 590
- US-A- 3 870 033
- US-A- 4 342 651
- US-A- 4 808 287
- US-A- 5 518 624

## Description

### OBJECT OF THE INVENTION

This invention is a water treatment system for haemodialysis, which has been devised with a view to improving the results that are yielded and the functions that are provided by the systems that are currently available, which are aimed at treating water for haemodialysis in hospitals.

The purpose of the invention is to provide a system by means of which it is possible to obtain a continuous supply of water to the ring to which the haemodialysis monitors are connected. Furthermore, it is equipped with the resources that enable it to control the quality of the water being treated, and this can be done either manually or automatically.

### BACKGROUND TO THE INVENTION

Two types of system are known for treating water for haemodialysis, one of which is the so-called in-line system and the other type of system is the one which involves tanks in which the water that is produced builds up.

The water treatment systems for dialysis are generally based upon the use of equipment for the prior disinfecting of the apparatus, followed by the use of inverse osmosis equipment by means of which the water is suitably treated, followed by the use of equipment or a ring that connects the haemodialysis monitors.

The problems that occur with the systems currently used, regardless of whether they are of the in-line type or those that are equipped with tanks in which the water produced builds up, lie in a series of drawbacks and disadvantages, such as the need to stop the equipment involved if the water supply is cut off, that is to say, they are unitary items of equipment in which any irregularity or problem makes it necessary to stop them and cut off the water supply.

Furthermore, the items of equipment are supplied directly by the water supply network, so if a failure affects the water supply network this clearly means that it is not possible to supply the equipment with water so it is necessary to switch it off.

At the very most, the water quality is controlled at the inverse osmosis outlet point, so any drop in the water quality level, for example, at the haemodialysis monitor connection ring, goes undetected, with the consequent problems that this can lead to.

Another drawback with the known systems is the lack of endotoxin retention and a lack of deionisers to carry out an optimum purification before the haemodialysis monitors are connected to the ring concerned.

The in-line systems are not equipped with resources that enable them to store the water produced, so this water is made to recirculate in the distribution ring and the surplus is made to flow back through the inverse osmosis circuit, so some of the water is wasted.

Therefore, on the basis of all these resource deficiencies affecting the known systems for treating water for haemodialysis, the bacteriological quality of the water as a whole and the physical-chemical quality are not optimum.

Different approaches have been made in order to obtain highly pure water for medical use. Thus, in US4342651, US5518624, US3870033, and EP752391 are disclosed apparatus with different combinations or sequences of means for the purification of water.

EP1234590 describes a water supply system suitable for preparing dialyses feed water, wherein the tank is foreseen before the reverse osmosis step, thus to the tank it arrives the prefiltered water and the leftover water from the dialysis process to feed the osmosis.

### DESCRIPTION OF THE INVENTION

The system that is the subject of this invention as defined in claim 1, combines the techniques of inverse osmosis and deionisation, making it possible to provide an uninterrupted high-quality water supply with high safety levels, on the basis of the demand required by the dialisation monitors.

To be specific, the system used by the invention is distinct because it is equipped with two production lines that can work together and alternate under normal operating conditions, and it can also work on an individual basis in the event of either of the two lines being affected by an operating problem, thereby ensuring that there is an ongoing supply of water as required.

The duplex nature of the equipment provides the system with two lines of inverse osmosis production, with the advantage that filtering resources are incorporated into these two lines; there is a prefiltering system to hold back the larger particles, reducing the concentration of iron and the hardness and removing the free chlorine, followed by a microfiltering process, which holds back the smaller particles.

The prefiltering system features a stainless steel mesh filter equipped with automatic cleaning, which holds back the larger particles, as well as a filter with a multimedia bed of quartz sand and anthracite; an iron removing filter, a decalcifier, whose use is also subject to the analysis of the inflowing water, and an active carbon filter; these filters operate in a completely automatic way, and the same applies to their cleaning or regeneration processes.

The microfiltering stage is carried out with the aid of a series of filter bags or cartridges of different grain sizes, with a view to increasing the working life of the membranes. After each microfiltering stage, a descaler is injected in, with a view to protecting the membranes from any possible residual scaling.

All of this takes place in each one of the two water conditioning lines, before going on in each case to the inverse osmosis stage in the strictest sense, for which there is also a duplex system; the water flows out in treated form and is then pumped to the distribution ring, which is original in the sense that it is provided with deionising, sterilising and endotoxin duplex filtering equipment; the sterilisation process is performed by means of an ultraviolet steriliser, and the endotoxins are filtered with the aid of an endotoxin retention filter.

The system is also equipped with a tank where the water can build up, so it can supply the inverse osmosis treatment lines even if the network water supply is cut off, in view of the fact that the water that builds up in this tank provided at the beginning has sufficient capacity to be able to supply water for 24 hours, a period that is more than enough to deal with a water network supply failure. Furthermore, the two treatment or water conditioning and inverse osmosis lines are supplemented with the same number of by-passes, either in a traditional inverse osmosis machine with its filtering chain, or via a chain of cartridge filters.

Another characteristic of the system that is the subject of the invention is the ongoing improvement of the water in the ring for recirculating to the dialysers, in view of the fact that although the water that is provided for the ring is already sterilised by means of a presterilisation system that is installed before the osmosis process, after having been deionised in the distribution ring, it is once again sterilised by ultraviolet ray, just in case any point on the water ring happens to be contaminated, the original aspect of this being that the deionisation process that takes place in that distribution ring, makes it possible to correct any water treatment caused by faulty operations or a failure affecting the preliminary equipment, or a temporary worsening of the quality of the inflow water for the hospital. Although the ultraviolet rays kill the bacteria, the viruses and other pathogenic organisms, the bodies of these dead organisms have to be removed from the water line, for the purpose of which we fit an endotoxin filter retention device after the steriliser.

The system also features four control points and safety levels that guarantee that the water that reaches the dialisation monitors complies with the desired quality specifications, and there is a control point that takes the form of a conductivity gauge and thermometer that are fitted behind the water supply point, thus controlling the quality of the water that supplies the system. There is a second control point that takes the form of another conductivity gauge fitted at the inverse osmosis outflow point, so as soon as the safety levels set are exceeded, visual and acoustic alarms go off that warn about the irregularity that is affecting operations on the line, and a change of line takes place. There is a third control point that also takes the form of a conductivity gauge, which is installed at the deioniser outflow point, and this third control point is responsible for the ongoing measurement of the water at the aforementioned deioniser, in such a way that as soon as the safety levels set are exceeded, visual and acoustic alarms go off that warn about the irregularity that is affecting operations on the line, and a change of line takes place. There is also a fourth control point, that also takes the form of a conductivity gauge which is fitted to the distribution ring return pipe, and this fourth control point is responsible for the ongoing measurement of the water before it flows back into the distribution ring, and as soon as the safety levels set are exceeded, visual and acoustic alarms go off that warn that the water has become contaminated during the dialysis process.

Safety where the way the system operates is concerned is controlled by ongoing checking, an automaton control and manual control.

That is to say, the system contains a checking system in its software that receives in real time, digital signals, logical states and alarms for the pressure in the pipes and filters, flow rates, conductivity, temperature, the situation in the electrovalves, the levels in the tanks, pressure units, etc, which it not only monitors, but also uses for taking corrective measures that restore the system to its normal state in the event of an irregularity occurring.

Furthermore, set points and programming are carried out on the automaton level, in such a way that under normal operation of the system these values are read from the software and they can be modified in real time. If the software becomes locked at any time, control over the system is returned to the automatons, which carry on working with the values that were originally set and the system carries on with the production without any interruptions.

If all or any of the automatons fail and they cannot be repaired immediately, the system passes on to manual control, the main measurement and control systems being duplicated in such a way that reading and manual handling can take place from the equipment itself.

The system will be backed up with a supervisor control system supervised by Personal Computer, with a view to guaranteeing reliability and tolerance to failures; this system is equipped with the following:

Monitoring and control. The main system display will show a flow diagram of all the items of equipment that form part of the facilities, together with the latest values for the variables that are sampled. Furthermore, it will also show, by colour changes, all the items of equipment that are operating at that particular time.

The application will be responsible for monitoring the entire treatment /purification process in real time.

Monitoring. Displaying important variables, start-up / shutdown of pumps, establishing which filters are operating, the positioning of all the valves, displaying the active route in the process, indicating which item of equipment is working, in those cases where there is redundancy, alarms and warnings for carrying out scheduled maintenance operations and alarms, if such operations have not been performed after the specified deadline has elapsed.

Set-point values. The set-point values are entered directly into the Personal Computer and these values are sent to the PLC that will be in charge of controlling the system; this information is recorded in the historical database.

Alarm management. The alarms that are detected are recorded and displayed, and they cannot be removed until the cause has disappeared.

Special operations. Cleaning the equipment with ozone requires an automatic sequence that although it is controlled by the PLC, is initiated by an operator from the Personal Computer. It is monitored and recorded.

Record of maintenance operations. The system is equipped with a record of all the maintenance operations that have been performed on the equipment.

Historical data file. As has already been pointed out, the system database records all the information.

System administration. Only authorised operators who have identified themselves beforehand, can change the set-point values. Queries can be restricted by requiring that those who wish to make queries be authorised to do so.

Remote notification of alarms. SMS messages. It is possible to inform about alarms by sending SMS messages to the mobile phone numbers of users who have been specified beforehand.

Remote monitoring. Monitoring Centre. This is the technology that is used for connecting purposes, the process enables the user to display in real time from any post that is connected to the process.

Disinfecting the entire water treatment system, not only the treated water circuit, using Ozone to do so.

Preventing and foreseeing microbiological contamination is the target, rather than merely correcting it after the event. If only the treated water circuit is disinfected, the items of prefiltering equipment are left without germicide treatment, which could lead to a major biological load before osmosis.

The prefiltering systems are items of equipment where the water stagnates, sometimes for long periods of time. Such environments are conducive to the proliferation of microbiological growth. This risk of contamination increases, if one considers that it is not uncommon in some hospitals to find concentrations of free chlorine in the water below levels that are advisable.

This proliferation of bacteria can have an adverse effect upon the way the inverse osmosis process works, because although it rejects 99% of the bacteria, a proportion remain on the surface of the membranes making them deteriorate and reducing their performance. Furthermore, it is possible that 1 % are passed on to the treated water circuit.

### DESCRIPTION OF THE DRAWINGS

One single page of plans is included with a view to supplementing the description that is being provided and in order to help to give a better working knowledge of the invention's characteristics, in accordance with an example of the realisation of a preferred practical embodiment thereof; these plans show a diagram for the water treatment system for dialysis carried out in accordance with the object of the invention, and they are merely illustrative, and in no way limiting.

### REALISATION OF A PREFERRED EMBODIMENT OF THE INVENTION

The above-mentioned figure shows how the system for the invention consists of three blocks defined by continuous and dotted lines, these blocks indicating references (1), (2) and (3), the first of which is a disinfecting system for the equipment using Ozone and includes an Ozone generator (4), a redox (5), an air drier (6), a recirculating pump (7), a Venturi ejector (8) and a redox gauge (9).

The whole assembly initially includes a tank (10) where the water builds up, the water flowing in from the water supply network, through the intake (11), which has been equipped with an electrovalve (12), and the tank is equipped with a level gauge (13).

The water coming from that tank (10) flows into the block (2) where the inverse osmosis takes place with filtering and sterilisation, and the inverse osmosis process is brought about by an item of duplex equipment, because there are two lines (14) and (15) with the same apparatus and devices, these two lines (14) and (15) forming an item of duplex equipment for treatment and inverse osmosis, which the water coming from the tank (10) flows into after having passed through a conductivity gauge (C) and a thermometer (T), the purpose of which are to control the supply water for this duplex inverse osmosis equipment.

The water is conditioned for both line (14) and line (15), and this is based upon a filtering process so that the water is conditioned and it can then be treated in the inverse osmosis circuit, both lines (14) and (15) being equipped with a piece of supply apparatus (16) that is responsible for supplying the facilities with water, absorbing the water loss that the line and the equipment causes, in such a way that after those pieces of supply apparatus (16), lines (14) and (15) are equipped with the respective filtering resources, which consists of a prefiltering stage followed by a microfiltering stage.

The prefiltering stage is composed of a stainless steel mesh filter with automatic cleaning (17), which holds back the largest particles, as well as a filter with a multimedia bed of quartz sand and anthracite (18) an iron removing filter (19), a decalcifier (20), the use of which, just like the iron removing filter (19) is subject to the analysis of the inflowing water, and this stage also has an active carbon filter (21).

The microfiltering stage is carried out with a series of filters (22), thick cartridges containing different grain sizes, so that the membranes last longer, and an anti-scaling injector (23) is placed after this series of filters (22); the anti-scaling injector (23) performs the final stage of the water conditioning process and is equipped with means for protecting the membranes from any residual scaling. The system is equipped with an ultraviolet steriliser (24) after this, from which the water flows through lines (14) and (15) to the inverse osmosis circuits (25), where optimum water is obtained for dialisation.

This water produced in one or other of these production and inverse osmosis lines (14) and (15), or what amounts to the same, in the circuits (25), flows onto the distribution ring (26) onto which one can connect the respective haemodialysis monitors, the above-mentioned distribution ring (26) forming part of the block (3) referred to at the beginning.

The water is pumped into this distribution ring (26) by a pumping unit (27) associated with a control panel (28), and the water flows into a duplex circuit for deionising, sterilising and filtering endotoxins, the deionising process being carried out with a deioniser (29) that is provided for each of the two lines on the circuit concerned, and then there are two ultraviolet sterilisers (30); the ends of both lines are equipped with a filter that holds back endotoxins (31).

The system consists of the aforementioned conductivity gauge and thermometer installed at the supply point, as well as a second conductivity gauge (C) fitted at the inverse osmosis outlet, a third conductivity gauge (C) fitted to each of the two lines where the deionising process takes place in the distribution circuit (26), and a fourth conductivity gauge (C) that is fitted to the return pipe for the aforementioned distribution ring (26).

Apart from the above-mentioned conductivity gauges (C), it also contains the respective thermometers (T), pressure gauges (P), pressure transducers and manometer (T-M), sample takers (TM), and valve regulators indicated by a symbol showing a circle with an inner blade, non-return valves, indicated by a symbol showing a square with a solid triangle inside it, and electrovalves, indicated by a symbol with a blade inside and a spiral form outside this symbol.

Under normal operating conditions the system is controlled by an automaton which receives the signals generated by the control and measurement instruments fitted in the equipment. The automaton is connected to a Personal Computer so that the value of the signals picked up by the automaton can be displayed and recorded, the alarms and the warning can be displayed and recorded and the set-point values can be entered.

The system is also equipped to be run manually, should a failure affect the automaton. The signals can also be processed without resorting to the automaton.

All the filters are equipped with differential manometers that enable the user to control the operating process and the cleaning process by pressure difference either through the automaton or manually. The way the lines operate and their cleaning process will both be governed by the automaton on the basis of the signals received (normal mode).

The duplex systems enable the user to receive an uninterrupted water supply on demand, invariably guaranteeing that the system operates both automatically and manually, independently of the equipment. To achieve this, each item of equipment is able to use either line (14) or line (15), thanks to a set of electrovalves governed by the automaton, or, thanks to a set of manual valves, if the automaton is not working.

In the system, the water quality is controlled at four different points, the first of which is the supply point, and is merely informative, so that the quality of the inflow water can be known at all times. The second point is at the inverse osmosis outlet, and it has two controls, the first uses a visual signal to inform about the advisability of changing osmosis lines, while the second uses a visual and acoustic signal to inform that that line cannot carry on producing, at which time the automaton changes lines (if the equipment were to be provided with membrane cleaning it would start or would indicate its initiation to recover the membrane(s)) and if it were manual, it would be necessary to manually change the line for the osmosis equipment. The third point is at the deioniser outlet, and it has two controls, the first uses a visual signal to inform about the advisability of changing the deionisation line, while the second uses a visual and acoustic signal to inform that that line cannot carry on producing, at which time the automaton changes lines (if the regeneration equipment were to begin or indicate its initiation to recover the resins) and if it were manual, it would be necessary to manually change the line for the deionisation equipment. The fourth one is positioned in the ring return pipe, in such a way that the visual and acoustic signal informs that the water is contaminated in the ring, as a result of a fault affecting the dialisation monitor connection points.

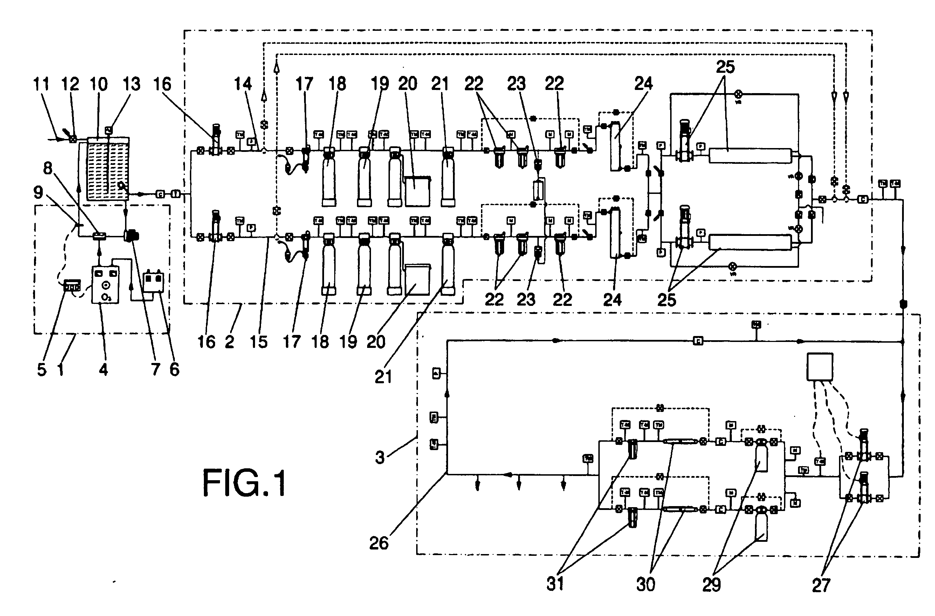

## Claims

1. A water treatment system for haemodialysis, comprising means for disinfecting the equipment using Ozone, as well as a circuit that purifies the water by inverse osmosis, and a distribution ring, to which the respective haemodialysis monitors are connected, **characterised by** the fact that the system is equipped with a tank (10) to collect the water flowing in from the supply network, which makes it possible to supply water to two parallel production lines (14) and (15) which are planned to work together, either alternately or individually, thereby guaranteeing an uninterrupted water supply, and the inverse osmosis process, in the event of the network water supply being cut off; each line being supplied by a device (16); each line comprising a filtering stage followed by a duplex inverse osmosis unit (25), further **characterised by** the fact that the lines are connected to a distribution circuit (26) foreseen after the inverse osmosis unit, to which the respective dialisation monitors are connected and said circuit comprising a pumping unit (27) and is also provided with a duplex circuit each being equipped with a deioniser (29), an ultraviolet steriliser (30) and a filter for holding back endotoxins (31).

2. A water treatment system for haemodialysis, according to Claim 1, **characterised by** the fact that the filtering stage that is located on each one of the lines (14) and (15), before the duplex circuit for inverse osmosis (25), is equipped with a prefiltering stage with a stainless steel mesh filter with automatic cleaning (17), as well as a filter with a multimedia bed of quartz sand and anthracite (18), an iron removing filter (19) and a decalcifier (20), followed by an active carbon filter (21); after this prefiltering stage there is a microfiltering stage, which consists of a series of filters (22), including an anti-scaling injector (23), and it is also provided with an ultraviolet steriliser (24) on each of the conditioning and treatment lines (14) and (15), before the duplex circuit for inverse osmosis (25).

3. A water treatment system for haemodialysis, according to any of claims 1-2, **characterised by** the fact that conductivity gauges (C) are provided to control conductivity in the water at all points, not only in the water supply coming from the storage tank (10), but also after the duplex circuit for inverse osmosis (25), as well as in each one of the lines in the distribution ring (26) and in the return pipe for the latter.

4. A water treatment system for haemodialysis, according to any of claims 1-3, **characterised by** the fact that it is equipped with thermometers (T), pressure gauges (P), pressure transducers and manometer (T-M), sample takers (TM) and regulating valves, manual valves, non-return valves and electrovalves, so that the system can operate in either automatic mode or manual mode.

5. A water treatment system for haemodialysis, according to any of claims 1-4, **characterised by** the fact that it establishes control by an automaton, which receives the signals generated by the measurement and control instruments installed, this automaton being connected to a Personal Computer so that the values of the signals picked up by the automaton can be displayed and recorded, the alarms and warnings can be displayed and recorded and the set-point values can be entered; and it is designed in such a way that the measurement instruments, as well as the valves and the rest of the components, can control the operation both manually and automatically.

## Patentansprüche

1. Eine Wasseraufbereitungsanlage für die Hämodialyse, die Mittel zur Desinfektion der Geräte durch Ozon sowie einen Kreislauf umfasst, der das Wasser durch Umkehrosmose reinigt und einen Verteilungsring, an den die jeweiligen Hämodialyse-Monitore angeschlossen sind, die sich **dadurch kennzeichnet, dass** die Anlage mit einem Behälter (10) zur Ausnahme des aus dem Versorgungsnetz einfließenden Wassers ausgestattet ist, was es ermöglicht, zwei parallele Produktionsleitungen (14) und (15) mit Wasser zu versorgen, die darauf ausgelegt sind, entweder abwechselnd oder einzeln zusammen zu arbeiten, wodurch eine ununterbrochene Wasserversorgung gewährleistet wird, und den Umkehrosmose-Prozess, wobei bei einer Unterbrechung des Wasser aus dem Versorgungsnetz jede Leitung durch eine Vorrichtung (16) versorgt wird; jede Leitung umfasst dabei eine Filterstufe gefolgt von einer Duplex-Umkehrosmose-Einheit (25), die sich weiter **dadurch kennzeichnet, dass** die Leitungen mit einem Verteilerkreis (26) verbunden sind, an den die jeweiligen Dialysemonitore angeschlossen sind und dass besagter Kreis eine Pumpeinheit (27) umfasst und auch mit einem Duplex-Kreis versehen ist, wobei jeder mit einem Entionisierer (29), einem UV-Sterilisator (30) und einem Filter zur Zurückhaltung von Endotoxinen (31) ausgerüstet ist.

2. Eine Wasseraufbereitungsanlage für die Hämodialyse gemäß Patentanspruch 1, die sich **dadurch kennzeichnet, dass** sich die Filterstufe an jeder der Leitungen (14) und (15) vor der Duplexverbindung für die Umkehrosmose (25) befindet, mit einer Vorfilterstufe mit einem Edelstahlgewebefilter mit automatischer Reinigung (17) sowie einem Filter mit Mehrschichtfilterbett aus Quarzsand und Anthrazit (18), einem Eisenentfernungsfilter (19) und einem Entkalker (20) ausgestattet ist, dem ein Aktivkohlefilter (21) folgt; nach dieser Vorfilterstufe folgt eine Mikrofiltrierstufe, die aus einer Reihe von Filtern (22) einschließlich eines Antiscaling-Injektors (23) besteht und ebenso mit einem UV-Sterilisator (24) an jeder der Aufbereitungs- und Behandlungsleitungen (14) und 15) vor dem Duplex-Kreislauf zur Umkehrosmose (25) versehen ist.

3. Eine Wasseraufbereitungsanlage für die Hämodialyse gemäß einem der Patentansprüche 1-2, die sich **dadurch kennzeichnet, dass** die Leitfahigkeitsmessgeräte (C) zur Kontrolle der Leitfähigkeit im Wasser an allen Stellen vorgesehen sind, und dies nicht nur in der Wasserversorgung aus dem Lagerbehälter (10), sonder auch nach dem Duplex-Kreislauf zur Umkehrosmose (25) sowie in jeder Leitung im Verteilerring (26) und in der Rücklaufleitung für diesen.

4. Eine Wasseraufbereitungsanlage für die Hämodialyse gemäß einem der Patentansprüche 1-3, die sich **dadurch kennzeichnet, dass** sie mit Thermometern (T), Druckprüfern (P), Druckmessumformern und Manometern (T-M), Probennehmern (TM) und Regelventilen, Rückschlagventilen und Magnetventilen ausgestattet ist, so dass die Anlage entweder im Automatik- oder im manuellen Betrieb arbeiten kann.

5. Eine Wasseraufbereitungsanlage für die Hämodialyse gemäß einem der Patentansprüche 1-4, die sich **dadurch kennzeichnet, dass** die Steuerung per SPS erfolgt, die die von den installierten Mess- und Kontrollinstrumenten erzeugten Signale erhält, wobei diese SPS an einen Rechner angeschlossen ist, so dass die Werte der von der SPS aufgenommenen Signale angezeigt und gespeichert werden können, wobei Alarmmeldungen und Warnung angezeigt und gespeichert werden können und die Sollwerte einzugeben sind; und sie ist so ausgelegt, dass die Messinstrumente sowie die Ventile und die übrigen Komponenten den Betrieb manuell oder automatisch steuern können.

## Revendications

1. Un système de traitement d'eau pour hémodialyse, qui comprend des moyens de désinfection de l'équipement en utilisant de l'ozone, ainsi qu'un circuit qui purifie l'eau par osmose inverse et un circuit de distribution auquel les moniteurs d'hémodialyse respectifs sont connectés, **caractérisé par le fait que** le système est équipé d'un réservoir (10) destiné à recueillir l'eau qui y pénètre en provenance du réseau d'alimentation, ce qui permet de fournir de l'eau à deux lignes de production parallèles (14) et (15) qui sont conçues pour travailler ensemble, soit de manière alternée soit individuellement, garantissant ainsi une alimentation ininterrompue en eau et le processus d'osmose inverse au cas où l'alimentation en eau de réseau serait coupée ; chaque ligne est alimentée par un dispositif (16) ; chaque ligne comprend un étage de filtration suivi d'une unité duplex d'osmose inverse (25) ; les lignes se caractérisent également **par le fait qu'**elles sont connectées à un circuit de distribution (26) auquel les moniteurs de dialyse respectifs sont connectés, le dit circuit comprenant une unité de pompage (27) et est également équipé d'un circuit duplex, chacune étant munie d'un déioniseur (29), d'un stérilisateur aux ultraviolets (30) et d'un filtre pour retenir les endotoxines (31).

2. Un système de traitement d'eau pour hémodialyse, conformément à la revendication 1, **caractérisé par le fait que** l'étage de filtration qui est situé sur chacune des lignes (14) et (15) avant le circuit duplex d'osmose inverse (25) est équipé d'un étage de préfiltration avec un filtre à mailles en acier inoxydable à nettoyage automatique (17) ainsi qu'un filtre avec un lit composé de sable de quartz et d'anthracite (18), un filtre de suppression de fer (19) et un décalcificateur (20), suivis d'un filtre au charbon actif (21) ; après cet étage de préfiltration, il y a un étage de microfiltration, qui consiste en une série de filtres (22) incluant un ïnjecteur anti-tartre (23), et il est également fourni avec un stérilisateur aux ultraviolets (24) sur chacune des lignes de conditionnement et de traitement (14) et (15), avant le circuit duplex d'osmose inverse (25).

3. Un système de traitement d'eau pour hémodialyse conformément aux revendications 1 et 2, **caractérisé par le fait que** des jauges de conductivité (C) sont fournies pour contrôler la conductivité dans l'eau à tous les points, non seulement dans l'alimentation en eau provenant du réservoir de stockage (10) mais aussi après le circuit duplex d'osmose inverse (25), ainsi que dans chacune des lignes du circuit de distribution (26) et dans le tuyau de retour de ce dernier.

4. Un système de traitement d'eau pour hémodialyse conformément aux revendications 1 à 3, **caractérisé par le fait qu'**il est équipé de thermomètres (T), de manomètres (P), de capteurs de pression et d'un manomètre (T-M), d'échantillonneurs (TM) et de vannes de régulation, de vannes manuelles, de clapets de non-retour et d'électrovalves, de sorte que le système puisse fonctionner en mode manuel ou en mode automatique.

5. Un système de traitement d'eau pour hémodialyse conformément aux revendications 1 à 4, **caractérisé par le fait qu'**il est contrôlé par un automate, qui reçoit les signaux générés par les instruments de mesure et de contrôle installés, cet automate étant connecté à un ordinateur personnel de sorte que les valeurs des signaux captés par l'automate puissent être affichées et enregistrées, que les alarmes et les avertissements puissent être affichés et enregistrés et que les points de consigne puissent être entrés ; et il est conçu de telle manière que les instruments de mesure ainsi que les vannes et les autres composants puissent contrôler l'opération à la fois manuellement et automatiquement.
